(19) Europäisches Patentamt
European Patent Office
Office européen des brevets

(11) **EP 4 501 993 A1**

(12) **EUROPEAN PATENT APPLICATION**
published in accordance with Art. 153(4) EPC

(43) Date of publication:
**05.02.2025 Bulletin 2025/06**

(21) Application number: 23788274.1

(22) Date of filing: 07.04.2023

(51) International Patent Classification (IPC):
*C08F 222/38* (2006.01)      *A61L 15/24* (2006.01)
*A61L 27/34* (2006.01)       *A61L 29/08* (2006.01)
*A61L 31/10* (2006.01)       *C08F 220/06* (2006.01)
*C08F 220/54* (2006.01)      *G02C 7/04* (2006.01)

(52) Cooperative Patent Classification (CPC):
A61L 15/24; A61L 27/34; A61L 29/08; A61L 31/10;
C08F 220/06; C08F 220/54; C08F 222/38;
G02C 7/04

(86) International application number:
**PCT/JP2023/014354**

(87) International publication number:
**WO 2023/199855 (19.10.2023 Gazette 2023/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 13.04.2022   JP 2022066091
30.01.2023   JP 2023011776

(71) Applicant: Toray Industries, Inc.
Tokyo 103-8666 (JP)

(72) Inventors:
• **TABARA, Saori**
  **Kamakura-shi, Kanagawa 248-8555 (JP)**
• **KITAGAWA, Rumiko**
  **Otsu-shi, Shiga 520-8558 (JP)**
• **NAKAMURA, Masataka**
  **Otsu-shi, Shiga 520-8558 (JP)**
• **KADOWAKI, Koji**
  **Otsu-shi, Shiga 520-8558 (JP)**

(74) Representative: **Prüfer & Partner mbB**
**Patentanwälte · Rechtsanwälte**
**Sohnckestraße 12**
**81479 München (DE)**

(54) **COPOLYMER, MEDICAL COMPOSITION AND COATED MEDICAL DEVICE, AND METHODS RESPECTIVELY FOR PRODUCING THOSE**

(57)   An object of the present invention is to provide a copolymer that is capable of imparting excellent biocompatibility to the surface of a medical device by a simple process and keeping the biocompatibility for a long period of time, a method for producing the copolymer, and the like. The present invention provides a copolymer containing the following (A), (B), and (C) as monomer components: (A) a compound having a carboxybetaine group and/or a sulfobetaine group; (B) a (meth)acrylic acid; and (C) an acrylamide derivative.

**Description**

TECHNICAL FIELD

The present invention relates to a copolymer, a medical composition, a coated medical device, and methods respectively for producing those.

BACKGROUND ART

Medical devices in direct contact with living bodies, such as contact lenses and stents, need to have biocompatibility. For ensuring safety and improving wearing feeling, the surfaces of contact lenses need to have biocompatibility such as water wettability, lubricity, and stain resistance. Stents are implanted medical devices that can be placed in bodies, which are considered to have the major problem of causing complications due to adhesion of thrombus, platelet, mucus, and the like. In particular, as for stents for respiration, there is a great clinical need for stents that can be used for a long period of time, with mucus adhesion suppressed and biocompatibility improved. As representative approaches for imparting excellent biocompatibility to the surface of a medical device, surface treatments for the medical device are known.

As surface treatment methods for medical devices, a method of coating laminated polymers that differ in properties from each other (Patent Document 1), a method of coating a polymer containing an amphoteric electrolyte compound (Patent Document 2), a method of coating a random copolymer of N-(meth)acryloyloxyethyl-N,N-dimethylammonium-$\alpha$-N-methylcarboxybetaine and butyl methacrylate (Patent Document 3), and the like are known.

PRIOR ART DOCUMENTS

PATENT DOCUMENTS

Patent Document 1: WO 2013/024801
Patent Document 2: National Publication of International Patent Application No. 2014-520191
Patent Document 3: Japanese Patent No. 4961133

SUMMARY OF THE INVENTION

PROBLEMS TO BE SOLVED BY THE INVENTION

Many of conventional surface treatment methods for medical devices, however, involve complicated operations, and moreover, the biocompatibility obtained by the surface treatments has sufficient durability at present.

Accordingly, an object of the present invention is to provide a copolymer that is capable of imparting excellent biocompatibility to the surface of a medical device by a simple process and keeping the biocompatibility even when the copolymer is exposed to a fictional stimulation, a method for producing the copolymer, and the like.

SOLUTIONS TO THE PROBLEMS

For achieving the object mentioned above, the present invention provides a copolymer containing the following (A), (B), and (C) as monomer components:

(A) a compound having a carboxybetaine group and/or a sulfobetaine group;
(B) a (meth)acrylic acid; and
(C) an acrylamide derivative.

EFFECTS OF THE INVENTION

According to the present invention, it is possible to provide a copolymer that is capable of imparting excellent biocompatibility to the surface of a medical device without complicated operations and keeping the biocompatibility even when the copolymer is exposed to frictional stimulation.

BRIEF DESCRIPTION OF THE DRAWINGS

Fig. 1 is a graph showing the amounts of mucin adhering to silicone hydrogel lenses.
Fig. 2 is a graph showing the amounts of mucin adhering to hydrogel lenses.
Fig. 3 is a graph showing the amounts of lysozyme adhering to silicone hydrogel lenses.
Fig. 4 is a graph showing the amounts of lysozyme adhering to hydrogel lenses.
Fig. 5 is a graph showing the amounts of mucin adhering to silicone hydrogel lenses.
Fig. 6 is a graph showing the amounts of mucin adhering to hydrogel lenses.
Fig. 7 is a graph showing the amounts of lysozyme adhering to silicone hydrogel lenses.
Fig. 8 is a graph showing the amounts of lysozyme adhering to hydrogel lenses.
Fig. 9 is a graph showing the amounts of mucin adhering to airway stents.

EMBODIMENTS OF THE INVENTION

A copolymer according to the present invention is a multicomponent copolymer (hereinafter, a "copolymer T") containing (A) a compound having a carboxybetaine group and/or a sulfobetaine group, (B) a (meth)acrylic acid, and (C) an acrylamide derivative as monomer components.

The copolymer according to the present invention is non-crosslinked and may be linear or branched, but is preferably linear from the viewpoint of ease of production. The copolymer according to the present invention may be any of a random copolymer, an alternating copolymer, a block copolymer, and a graft copolymer. From the viewpoint of ease of production, a random copolymer is preferred.

The "monomer component" as a raw material for the copolymer according to the present invention refers to a low molecular weight compound that can be polymerized and/or crosslinked with the use of a photopolymerization initiator and/or a thermal polymerization initiator. In addition, the fact that the copolymer contains the (A), (B), and (C) as monomer components means that the copolymer contains a structure derived from the monomer components (A), (B), and (C).

Examples of the (A) "compound having a carboxybetaine group and/or a sulfobetaine group" (hereinafter, a "monomer component (A)"), which is one of the monomer components, include 2-[[2-(methacryloyloxy)ethyl]dimethylammonio]acetate, 3-[[2-(methacryloyloxy)ethyl]dimethylammonio]propionate (hereinafter, "CBMA"), 3-[(3-acrylamidopropyl)dimethylammonio]propionate (hereinafter, "CBAA"), 3-[[2-(methacryloyloxy)ethyl]dimethylammonio]propane-1-sulfonate (hereinafter, "SBMA"), 4-[[2-(methacryloyloxy)ethyl]dimethylammonio]butane-1-sulfonate, 3-[[2-(acryloyloxy)ethyl]dimethylammonio]propane-1-sulfonate (SPDA), bis[2-(methacryloyloxy)ethyl](methyl)ammonio]propane-1-sulfonate, 3-[(3-methacrylamidopropyl)dimethylammonio]propane-1-sulfonate, 4-[(3-methacrylamidopropyl)dimethylammonio]butane-1-sulfonate, or 3-[(3-acrylamidopropyl)dimethylammonio]propane-1-sulfonate. From the viewpoint of the non-adsorption property of protein, CBMA or SBMA is preferred.

The (B) "(meth)acrylic acid" (hereinafter, a "monomer component (B)"), which is one of the monomer components, refers to a methacrylic acid and/or an acrylic acid (hereinafter, "AA"). From the viewpoint of polymerization stability, an acrylic acid is preferred.

Examples of the (C) "acrylamide derivative" (hereinafter, a "monomer component (C)"), which is one of the monomer components, include acrylamide, N-isopropylacrylamide, N,N-dimethylacrylamide (hereinafter, "DMAA"), N,N-diethylacrylamide, N-methyl-N-ethylacrylamide, or N,N-di(hydroxyethyl) acrylamide. From the viewpoint of polymerization stability, DMAA is preferred.

As for the proportions of the monomer component (A), monomer component (B), and monomer component (C) in the copolymer according to the present invention, the monomer component (A) is preferably 1 to 40 mol%, the monomer component (B) is preferably 1 to 35 mol%, and the monomer component (C) is preferably 25 to 98 mol%.

The proportion of the monomer component (A) in the copolymer according to the present invention is more preferably 1 to 30 mol%, still more preferably 5 to 25 mol%.

The proportion of the monomer component (B) in the copolymer according to the present invention is more preferably 1 to 25 mol%, still more preferably 5 to 15 mol%.

The proportion of the monomer component (C) in the copolymer according to the present invention is more preferably 45 to 98 mol%, still more preferably 60 to 90 mol%.

A method for producing a copolymer according to the present invention includes a polymerization step of polymerizing a monomer containing (A) a compound having a carboxybetaine group and/or a sulfobetaine group, (B) a (meth)acrylic acid, and (C) an acrylamide derivative to obtain the copolymer.

For polymerizing and/or crosslinking each of the monomer components in the polymerization step, a polymerization initiator such as a thermal polymerization initiator or a photopolymerization initiator can be used.

Examples of the thermal polymerization initiator include peroxides or azo compounds. For the thermal poly-

EP 4 501 993 A1

merization initiator, a thermal polymerization initiator that has optimal decomposition characteristics at a desired reaction temperature may be selected, and generally, an azo compound or a peroxide with a 10 hour half-life temperature of 40 to 120°C is preferred.

More specific examples thereof include 2,2'-azobis(isobutyronitrile) (AIBN), 2,2'-azobis(2-methylbutyronitrile), 2,2'-azobis(2,4-dimethylvaleronitrile), 1,1'-azobis(cyclohexane-1-carbonitrile), 2,2'-azobis[2-(2-imidazoline-2-yl)propane], 2,2'-azobis(2-methylpropionamidine)dihydrochloride (Hereinafter, "V-50"), 2,2'-azobis[N-(2-carboxyethyl)-2-methylpropionamidine], 2,2'-azobis[2-methyl-N-(2-hydroxyethyl)propionamide], 2,2'-azobis(N-butyl-2-methylpropionamide), 2,2'-azobis(2-methylpropionic acid)dimethyl, 4,4'-azobis(4-cyanovaleric acid), tert-butyl hydroperoxide, cumene hydroperoxide, di-tert-butyl peroxide, or benzoyl peroxide.

Examples of the photopolymerization initiator include carbonyl-based compounds such as aromatic $\alpha$-hydroxyketone, alkoxy benzoin, acetophenone, an acylphosphine oxide, or a bisacylphosphine oxide, tertiary amines + diketones, peroxides, azo compounds, sulfur compounds, halogen compounds, or metal salts.

More specific examples thereof include 1-hydroxycyclohexyl phenyl ketone, 2-hydroxy-2-methyl-1-phenyl-propan-1-one, bis(2,6-dimethoxybenzoyl)-2,4,4-trimethylpentylphosphine oxide (DMBAPO), bis(2,4,6-trimethylbenzoyl)-phenylphosphine oxide ("IRGACURE" (registered trademark) 819), 2,4,6-trimethylbenzyldiphenylphosphine oxide, 2,4,6-trimethylbenzoyldiphenylphosphine oxide, benzoin methyl ether, or a combination of camphorquinone and ethyl 4-(N,N-dimethylamino)benzoate.

These polymerization initiators may be used alone or in combination, but for obtaining a copolymer that has a desired molecular weight while appropriately promoting the polymerization or crosslinking of the monomer components, the proportion of the polymerization initiator in the polymerization mixture is preferably 5% by mass or less.

In this regard, the "polymerization mixture" refers to a mixture obtained by dissolving, in a solvent, the respective monomer components for obtaining a copolymer, a polymerization initiator, and a chain transfer agent that may be added, if necessary.

Examples of the solvent for obtaining the polymerization mixture include water, an alcohol-based solvents such as methanol, ethanol, propanol, 2-propanol, butanol, 2-butanol, tert-butanol, tert-amyl alcohol, 3,7-dimethyl-3 octanol, ethylene glycol, diethylene glycol, triethylene glycol, tetraethylene glycol, or polyethylene glycol; aromatic hydrocarbon-based solvents such as benzene, toluene, or xylene; aliphatic hydrocarbon-based solvents such as hexane, heptane, octane, decane, petroleum ether, kerosene, ligroin, or paraffin; alicyclic hydrocarbon-based solvents such as cyclopentane, cyclohexane, or ethylcyclohexane; ketone-based solvents such as acetone, methyl ethyl ketone, or methyl isobutyl ketone; ester-based solvents such as ethyl acetate, butyl acetate, amyl acetate, ethyl lactate, methyl benzoate, or ethylene glycol diacetate; or ether-based solvents such as diethyl ether, tert-butyl methyl ether, tetrahydrofuran, dioxane, ethylene glycol dimethyl ether, diethylene glycol dimethyl ether, triethylene glycol dimethyl ether, tetraethylene glycol dimethyl ether, or polyethylene glycol dimethyl ether. These solvents may be used alone or in combination. From the viewpoint of being less likely to inhibit radical polymerization, the solvent is preferably water, methanol, tert-butanol, tert-amyl alcohol, 3,7-dimethyl-3-octanol, tetrahydrofuran, or a mixed solvent thereof, more preferably water.

The total proportion of the monomer components in the polymerization mixture is preferably 10 to 80% by mass, more preferably 15 to 65% by mass for obtaining a copolymer that has a desired molecular weight while appropriately suppressing runaway due to polymerization heat.

In the polymerization step, the polymerization reaction is allowed to proceed by heating the polymerization mixture, thereby providing a desired copolymer.

The reaction temperature in the polymerization step may be selected depending on the decomposition temperature of the polymerization initiator, the boiling points of the solvent and monomers, the reaction time, and the like, but is preferably 20°C to 180°C, more preferably 50°C to 150°C, still more preferably 60 to 120°C for obtaining a desired copolymer in good yield.

The reaction time in the polymerization step may be selected depending on the types and concentrations of the polymerization initiator and monomers to be used, the reaction temperature, and the like, but is preferably 1 to 48 hours, more preferably 2 to 24 hours, still more preferably 3 to 12 hours for obtaining a desired copolymer in good yield.

The weight average molecular weight (hereinafter, "Mw") of the copolymer according to the present invention is preferably 1,000 to 5 million, more preferably 5,000 to 3 million, still more preferably 10,000 to 1 million, for obtaining suitable lubricity while ensuring that the viscosity (operability) of the solution is moderate.

A coated medical device according to the present invention is obtained by at least partially coating the surface of a medical device with the copolymer according to the present invention.

The material of the medical device may be any of a water-containing substrate such as a hydrogel (including a silicone hydrogel), an acrylic resin such as a polymethyl methacrylate, a silicone substrate having a siloxane bond, a metal such as aluminum, or a non-water-containing substrate such as glass.

Examples of the medical device includes an ophthalmic lens, a skin covering material, a wound covering material, a skin protective material, a drug carrier for skin, an infusion tube, a gas transport tube, a drainage tube, a blood circuit, a covering tube, a catheter, a stent, a sheath biosensor chip, a heart-lung machine, or an endoscope covering material. Among these examples, a medical device selected from an ophthalmic lens and a stent is preferred, because of a high demand for the effects of the present invention.

Examples of ophthalmic lenses include contact lenses such as soft contact lenses, hard contact lenses and hybrid contact lenses, scleral lenses, intraocular lenses, artificial cornea, corneal inlays, corneal onlays, or eyeglass lenses. Among these examples, contact lenses are preferred.

As the stent, a stent for a respiratory organ is preferred. The respiratory organ in the present invention is a generic term for organs related to respiration, and examples thereof include an airway, an oral cavity, a nasal cavity, a larynx, a trachea, a bronchus, a bronchiole, and a lung. A stent according to an embodiment of the present invention is a stent for a respiratory organ, and is preferably a stent for an airway, a trachea, a bronchus, or a lung.

A medical composition according to the present invention includes the copolymer according to the present invention and a solvent. The medical composition according to the present invention is used for at least partially coating the surface of a medical device with the copolymer according to the present invention to obtain a coated medical device according to the present invention.

A method for producing the medical composition according to the present invention includes a preparation step of mixing the copolymer according to the present invention and a solvent to obtain the medical composition.

Examples of the solvent for use in the preparation step include the same solvents as those for obtaining the "polymerization mixture" mentioned above.

The proportion of the copolymer according to the present invention in the medical composition according to the present invention is preferably 0.0001 to 30.0000% by mass, more preferably 0.0010 to 20.0000% by mass, still more preferably 0.0050 to 15.0000% by mass, for increasing the viscosity (operability) of the solution while ensuring that the thickness of the coating layer is moderate.

The surface of a medical device can be coated with the copolymer according to the present invention by physically bringing the medical composition according to the present invention into contact with the surface of the medical device. More specifically, the method for producing a coated medical device includes a coating step of bringing the medical composition according to the present invention into contact with the surface of a medical device to obtain the coated medical device. For example, to explain, as an example, a case where the medical device is an ophthalmic lens, the medical composition according to the present invention and the ophthalmic lens are enclosed in a container to be brought into contact with each other, and subjected to a heat treatment (coating step), thereby allowing the surface of the ophthalmic lens to be coated with the copolymer according to the present invention.

Examples of the approach for the heating operation include a high-pressure steam method, irradiation with electromagnetic waves such as visible light, infrared rays, or microwaves, an electrothermal method, an electromagnetic induction heating method, a dry heat method, and a flame method, and from the viewpoint of enhancing the water wettability and the lubricity and reducing the manufacturing process, a high-pressure steam method with an autoclave used is preferred.

The heating temperature in the heating operation is preferably 60 to 200°C, more preferably 80 to 180°C, still more preferably 100 to 170°C, particularly preferably 110 to 150°C, for obtaining a coated medical device surface that exhibits favorable water wettability and lubricity, while maintaining the strength of the medical device itself.

The heating time in the heating operation is preferably 5 to 600 minutes, more preferably 10 to 400 minutes, still more preferably 15 to 300 minutes, for obtaining a coated medical device surface that exhibits favorable water wettability and lubricity, while maintaining the strength of the medical device itself.

The initial pH of the medical composition according to the present invention is preferably 2.0 to 6.0, because the composition has no turbidity produced, thus providing a medical device that has favorable transparency and a surface with favorable water wettability and lubricity. The initial pH is more preferably 2.2 to 5.0, still more preferably 2.4 to 4.5, particularly preferably 2.5 to 4.0.

The pH of the medical composition according to the present invention can be adjusted, for example, by adding an acidic substance. Examples of the acidic substance include an acetic acid, a citric acid, a formic acid, an ascorbic acid, a trifluoromethanesulfonic acid, a methanesulfonic acid, a nitric acid, a sulfuric acid, a phosphoric acid, or a hydrochloric acid. The citric acid, ascorbic acid, or sulfuric acid, which has low volatility and high safety for living bodies, is preferred. In addition, for facilitating fine adjustment of the pH, a buffer may be added to the medical composition according to the present invention.

Examples of the buffer include a boric acid, borates such as a sodium borate; a citric acid, citrates such as a potassium citrate; bicarbonates such as a sodium bicarbonate; phosphates such as $Na_2HPO_4$, $NaH_2PO_4$, or $KH_2PO_4$); TRIS (tris(hydroxymethyl)aminomethane), 2-bis(2-hydroxyethyl)amino-2-(hydroxymethyl)-1,3-propanediol, bis-aminopolyol, triethanolamine, ACES (N-(2-acetamide)-2-aminoethanesulfonic acid), BES (N,N-bis(2-hydroxyethyl)-2-aminoethanesulfonic acid), HEPES (4-(2-hydroxyethyl)-1-piperazineethanesulfonic acid), MES (2-(N-morpholino)ethanesulfonic acid), MOPS (3-[N-morpholino]-propanesulfonic acid), PIPES (piperazine-N,N'-bis(2-ethanesulfonic acid)), or TES (N-[tris(hydroxymethyl)methyl]-2-aminoethanesulfonic acid), or salts thereof. The proportion of the buffer in the medical composition according to the present invention is preferably 0.001 to 2.000% by mass, more preferably 0.010 to 1.000% by mass, still more preferably 0.050 to 0.300% by mass, for achieve desired pH.

The pH of the medical composition according to the present invention may vary in the coating step such as a heating operation. For example, the pH of the medical composition immediately after the heating operation (before the pH adjustment) is preferably 2.0 to 6.5, more preferably 2.2 to 5.9, still more preferably 2.3 to 5.5, particularly preferably 2.4 to 5.0, for optimizing the physical properties of the coated medical device obtained.

The coated medical device obtained by the coating step mentioned above may be further subjected to another treatment. In this regard, examples of the other treatment include a treatment of performing the same coating step as mentioned above again, a treatment of performing the same heating operation, with a solvent, a buffer solution, or the like containing no copolymer according to the present invention in contact with the surface of the coated medical device, radiation irradiation, an LbL treatment (Layer by Layer treatment) of coating with polymer materials with opposite charges alternately in a layer-by-layer manner, a crosslinking treatment with metal ions, or a chemical crosslinking treatment.

The pH of the buffer solution of the buffer solution for use in the heat treatment is preferably physiologically acceptable 6.3 to 7.8, more preferably 6.5 to 7.6 or more, still more preferably 6.8 to 7.4.

EXAMPLES

Hereinafter, the present invention will be specifically described with reference to examples and the like, but the present invention is not limited thereto. First, analysis methods and evaluation methods in examples and the like will be described.

Further, the composition of the phosphate buffer solution used was as follows. KCl: 0.2 g/L, $KH_2PO_4$: 0.2 g/L, NaCl: 8.0 g/L, $Na_2HPO_4$: 1.2 g/L, EDTA: 0.5 g/L.

(1) Measurement of Mw and number average molecular weight (Mn) of polymer

The molecular weight of the polymer was measured with the use of a Prominence GPC system (manufactured by Shimadzu Corporation). The apparatus configuration was as follows.

Pump: LC-20AD, autosampler: SIL-20AHT, column oven: CTO-20A, detector: RID-10A, column: GMPWXL (manufactured by Tosoh Corporation; inner diameter 7.8 mm × 30 cm, particle diameter 13 pm). The measurement was carried out at a flow rate of 0.5 mL/min for a measurement time of 30 minutes with the use of water/methanol = 1/1 (with 0.1N lithium nitrate added) as an eluent. Further, the sample concentration was adjusted to 0.1% by mass, and the sample injection amount was adjusted to 20 μL. The calibration curve is created with the use of a PEG/PEO standard sample (manufactured by Agilent Technologies, Inc.; 0.1 to 1500 k) .

(2) Water wettability (liquid film retention time)

The coated medical device or the medical device (hereinafter, "test sample") was washed with the use of the phosphate buffer solution at room temperature, then immersed in the phosphate buffer solution at room temperature for 24 hours or longer, and allowed to stand. The test sample (hereinafter, "test sample S") was pulled up from the phosphate buffer solution, and held in the air such that the longitudinal direction of the sample was the direction of gravity. When the test sample is shaped in a lens, the test sample was held in the air such that the diameter direction of the circle formed by the edge of the lens was the gravity direction. The time from the time point of starting to hold the test sample S in the air until a breakage of a part of the liquid film covering the surface of the test sample S was visually observed, and the average value for N = 3 was determined in accordance with the following criteria. It is to be noted that "the breakage of a part of the liquid film" refers to a state in which the liquid membrane on the surface of the test sample S becomes incapable of maintaining the shape, thereby causing a part of the surface of the test sample S to be uncovered with the liquid film.

A: The liquid film on the surface is retained for 20 seconds or longer.
B: The liquid film on a surface is broken within 15 seconds or longer and shorter than 20 seconds.
C: The liquid film on a surface is broken within 5 seconds or longer and shorter than 15 seconds.
D: The liquid film on a surface is broken within 1 second or longer and shorter than 5 seconds.
E: The liquid film on a surface is broken instantly (within 1 second).

(3) Lubricity

When the test sample S pulled up from the phosphate buffer solution was rubbed five times with a human finger, a sensory evaluation was performed for N = 1, and determined in accordance with the following criteria.

A: Excellent lubricity (the finger slides so as to flow on the surface of the test sample S, without feeling any resistance).

B: Somewhat good lubricity (about the middle between A and C).
C: Moderate lubricity (the finger slides on the surface of the test sample S, almost without feeling any resistance).
D: Almost no lubricity (about the middle between C and E) .
E: No lubricity (the finger fails to slide easily on the surface of the test sample S, while felling large resistance).

(4) Water content

The surface of the test sample S pulled up from the phosphate buffer solution was wiped with a wiping cloth "Kimwipe" (registered trademark) (manufactured by NIPPON PAPER CRECIA CO., LTD.), and then, the mass (Ww) of the substrate was measured. Thereafter, the test sample S was dried at 60°C for 2 hours in a vacuum dryer, and then, the mass (Wd) thereof was measured. From these masses, the water content of the test sample was calculated by the following formula (1). The case where the obtained value was less than 1% was determined to be equal to or less than the measurement limit, and described as "less than 1%".
The average value for N = 3 was defined as the water content.

$$\texttt{Water content (\%) of test sample} = 100 \times (Ww - Wd)/Ww$$

$$\cdots \texttt{Formula (1)}$$

(5) Friction coefficient

The friction coefficient of the surface of the test sample S wetted with the phosphate buffer solution was measured under the following conditions, and the average value for N = 5 was defined as the friction coefficient.

Apparatus: Friction tester KES-SE (manufactured by KATO TECH CO., LTD.)
Friction SENS: H
Measurement SPEED: 2 × 1 mm/sec
Friction load: 44 g.

(6) Lipid adhesion amount

Into a 20 cc screw tube, 0.03 g of methyl palmitate, 10 g of pure water, and the test sample were put. The screw tube was shaken for 3 hours under the condition of 37°C and 165 rpm. After the shaking, the test sample in the screw tube was scrubbed and washed with the use of tap water at 40°C and a household liquid detergent "Mama Lemon" (registered trademark) (manufactured by Lion Corporation). The washed test sample was put into a screw tube containing the phosphate buffer solution, and stored in a refrigerator at 4°C for 1 hour. Thereafter, the test sample was visually observed, a cloudy part thereof, if any, was determined to be a part with the methyl palmitate adhering, and the area of the part with the methyl palmitate adhering with respect to the entire surface of the sample was observed.

(7) Mucin adhesion amount

When the test sample was a contact lens, a test piece of 5 mm in width (minimum part) and 14 mm in length was cut out with the use of a blanking die (hereinafter, "sample P"). When the test sample was a stent, a test piece of 4 mm in diameter was cut out with the use of a blanking die (hereinafter, "sample K"). With the use of Mucin, Bovine Submaxillary Gland (manufactured by Millipore Corporation; catalog number 499643) as a mucin, a mucin aqueous solution with a concentration of 0.1% by mass was prepared, and the sample P or the sample K was immersed therein under the condition of 37°C for 22 hours. Thereafter, the amount of mucin adhering to the sample P or the sample K was quantified by a BCA (bicinchoninic acid) protein assay method, and the average value for N = 3 was defined as the mucin adhesion amount.

(8) Lysozyme adhesion amount

With the use of a lysozyme derived from egg white (manufactured by FUJIFILM Wako Pure Chemical Corporation; catalog number 127-06724) as a lysozyme, a 0.1% by mass lysozyme aqueous solution was prepared, and the sample P was immersed therein under the condition of 37°C for 22 hours. Thereafter, the amount of lysozyme adhering to the sample P was quantified by a BCA protein assay method, and the average value for N = 3 was defined as the lysozyme adhesion amount.

[Example 1]

Into a 500 mL four-necked flask, 2.29 g (10 mmol) of CBMA (manufactured by Tokyo Chemical Industry Co., Ltd.), 0.72 g (10 mmol) of AA (manufactured by FUJIFILM Wako Pure Chemical Corporation), 7.93 g (80 mmol) of DMAA (manufactured by FUJIFILM Wako Pure Chemical Corporation), 6.8 mg (0.025 mmol) of V-50 (manufactured by FUJIFILM Wako Pure Chemical Corporation) as a polymerization initiator, and 98.49 g of purified water (manufactured by KOGA Chemical Mfg Co., Ltd.) were added, and the flask was equipped with a digital thermometer, a Dimroth cooling tube with a three-way cock attached, and a sealer with stirring blades. Under irradiation with ultrasonic waves, a cycle of suctioning the four-necked flask to a pressure of 10 mmHg and then flushing with nitrogen was repeated five times to remove oxygen dissolved in the mixed solution. Subsequently, the mixed solution was allowed to react at 90°C for 4 hours on an oil bath while stirring the solution, and then the four-necked flask was pulled up from the oil bath and air-cooled to obtain 109.44 g of a copolymer $T_1$. The properties of the obtained copolymer $T_1$ were as shown in Table 1.

[Example 2]

Except for using 2.79 g (10 mmol) of SBMA (manufactured by Tokyo Chemical Industry Co., Ltd.) in place of the CBMA, the same operations as in Example 1 were carried out to obtain 114.45 g of a copolymer $T_2$. The properties of the obtained copolymer $T_2$ were as shown in Table 1.

[Comparative Example 1]

Except for using 2.95 g (10 mmol) of 2-(methacryloyloxy)ethyl 2-(trimethylammonio)ethyl phosphate (hereinafter, "MPC"; manufactured by Tokyo Chemical Industry Co., Ltd.) in place of the CBMA, the same operations as in Example 1 were carried out to obtain 116.04 g of a copolymer $T_{C1}$ as the copolymer T. The properties of the obtained copolymer $T_{C1}$ were as shown in Table 1.

[Comparative Example 2]

Except for using N-vinylpyrrolidone (hereinafter, "NVP"; manufactured by Tokyo Chemical Industry Co., Ltd., 1.11 g, 10 mmol) in place of the CBMA, the same operations as in Example 1 were carried out to obtain 97.63 g of a copolymer $T_{C2}$. The properties of the obtained copolymer $T_{C2}$ were as shown in Table 1.

[Table 1]

|  | Copolymer T | Constituent monomer | Polymerization molar ratio | Mw | Mw/Mn |
|---|---|---|---|---|---|
| Example 1 | Copolymer $T_1$ | CBMA/AA/DMAA | 10/10/80 | 391k | 2.59 |
| Example 2 | Copolymer $T_2$ | SBMA/AA/DMAA | 10/10/80 | 497k | 3.25 |
| Comparative Example 1 | Copolymer $T_{C1}$ | MPC/AA/DMAA | 10/10/80 | 428k | 3.21 |
| Comparative Example 2 | Copolymer $T_{C2}$ | NVP/AA/ DMAA | 10/10/80 | 501k | 5.15 |

[Example 3]

As a medical device A, a commercially available silicone hydrogel lens "Acuvue Oasys" (registered trademark; manufactured by Johnson & Johnson K.K.) containing a polyvinylpyrrolidone and a silicone as main components was used. The copolymer $T_1$ was added to the phosphate buffer solution so as to have a concentration of 0.3% by mass, and the pH was adjusted to 3.0 with the use of a citric acid. Into a glass vial, 3 mL of the prepared solution was transferred, and the medical device A was immersed therein, and then heated in an autoclave at 121°C for 30 minutes. The heated medical device was washed with the phosphate buffer solution for 30 seconds, then put in a new phosphate buffer solution, and further heated in an autoclave at 121°C for 30 minutes to obtain a coated medical device.

[Example 4]

Except for using the copolymer $T_2$ in place of the copolymer $T_1$, the same operations as in Example 3 were carried out to obtain a coated medical device.

[Example 5]

As a medical device M, a commercially available hydrogel lens "Medalist" (registered trademark; manufactured by Baush & Lomb) containing N-vinylpyrrolidone and 2-hydroxyethyl methacrylate (2-HEMA) as main components. Except for using the medical device M in place of the medical device A, the same operations as in Example 3 were carried out to obtain a coated medical device.

[Example 6]

Except for using the copolymer $T_2$ in place of the copolymer $T_1$, the same operations as in Example 5 were carried out to obtain a coated medical device.

[Comparative Example 3]

Except for using the copolymer $T_{C1}$ in place of the copolymer $T_1$, the same operations as in Example 3 were carried out to obtain a coated medical device.

[Comparative Example 4]

Except for using the copolymer $T_{C2}$ in place of the copolymer $T_1$, the same operations as in Example 3 were carried out to obtain a coated medical device.

[Comparative Example 5]

Except for using the copolymer $T_{C1}$ in place of the copolymer $T_1$, the same operations as in Example 5 were carried out to obtain a coated medical device.

[Comparative Example 6]

Except for using the copolymer $T_{C2}$ in place of the copolymer $T_1$, the same operations as in Example 5 were carried out to obtain a coated medical device.

[Comparative Example 7]

The medical device A was evaluated without performing the coating step.

[Comparative Example 8]

The medical device M was evaluated without performing the coating step.

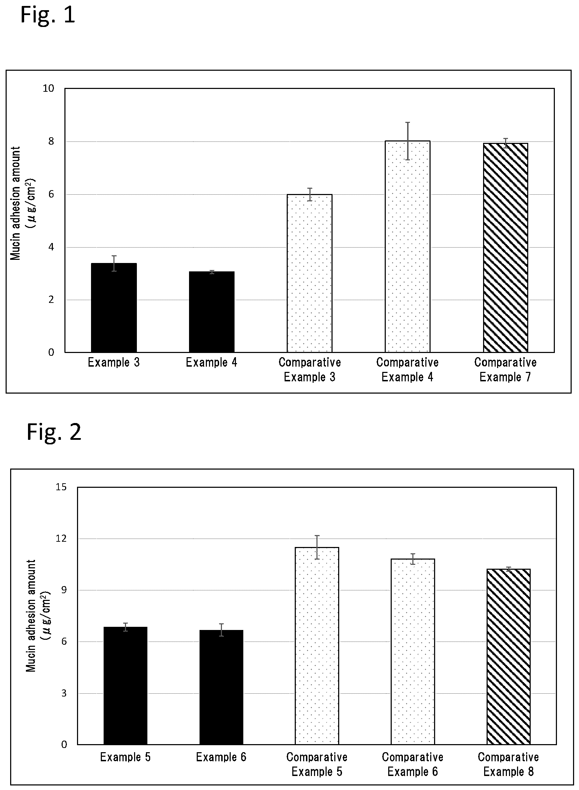

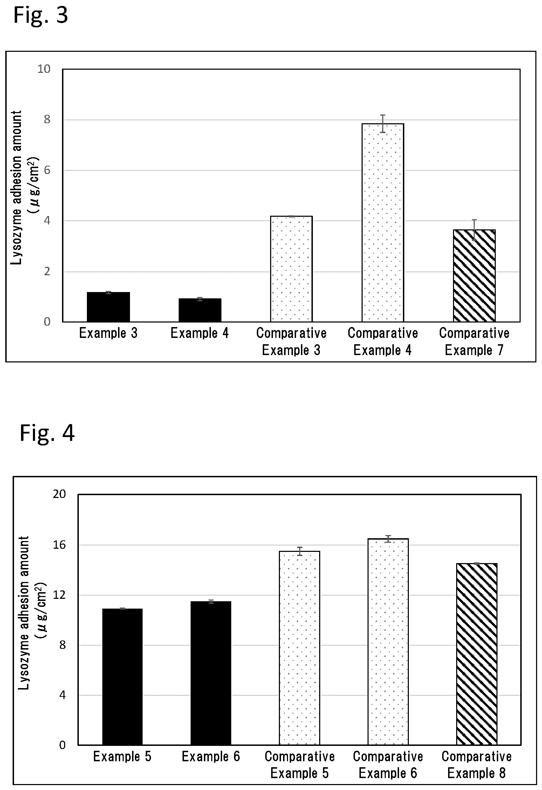

The evaluation results for each of the coated medical devices obtained in Examples 3 to 6 and Comparative Examples 3 to 8 are shown in Table 2 and Table 3 and Figs. 1 to 4.

[Table 2]

| | Copolymer T | Medical device | Initial pH | pH after heat treatment | Water content of device (%) |
|---|---|---|---|---|---|
| Example 3 | Copolymer $T_1$ | A | 3 | 3.2 | 35 |
| Example 4 | Copolymer $T_2$ | A | 3 | 3.3 | 35 |
| Example 5 | Copolymer $T_1$ | M | 3 | 3.2 | 58 |
| Example 6 | Copolymer $T_2$ | M | 3 | 3.3 | 57 |
| Comparative Example 3 | Copolymer $T_{C1}$ | A | 3 | 3.1 | 35 |
| Comparative Example 4 | Copolymer $T_{C2}$ | A | 3 | 3.5 | 35 |
| Comparative Example 5 | Copolymer $T_{C1}$ | M | 3 | 3.2 | 57 |
| Comparative Example 6 | Copolymer $T_{C2}$ | M | 3 | 3.5 | 57 |
| Comparative Example 7 | - | A | No | No | 35 |

(continued)

|  | Copolymer T | Medical device | Initial pH | pH after heat treatment | Water content of device (%) |
|---|---|---|---|---|---|
| Comparative Example 8 | - | M | No | No | 58 |

[Table 3]

|  | Liquid film retention time (second) | Liquid film retention time 2 hours after scrubbing and washing (sec) | Lubricity | Friction coefficient | Lipid adhesion |
|---|---|---|---|---|---|
| Example 3 | A (120) | A (59) | A | 0.127 | No adhesion |
| Example 4 | A (120) | A (77) | A | 0.134 | No adhesion |
| Example 5 | A (120) | A (45) | B | 0.207 | No adhesion |
| Example 6 | A (120) | A (61) | B | 0.275 | No adhesion |
| Comparative Example 3 | D (4) | E (0) | D | 0.17 | No adhesion |
| Comparative Example 4 | A (120) | A (55) | A | 0.125 | Partial adhesion |
| Comparative Example 5 | C (7) | E (0) | D | 0.489 | No adhesion |
| Comparative Example 6 | A (120) | A (48) | A | 0.109 | No adhesion |
| Comparative Example 7 | B (16) | E (0) | C | 0.182 | Adhesion |
| Comparative Example 8 | D (3) | E (0) | D | 0.427 | Adhesion to whole surface |

[Example 7]

Except for using 4.59 g (20 mmol) of the CBMA, 0.72 g (10 mmol) of the AA, 6.94 g (70 mmol) of the DMAA, and purified water (90.21 g), the same operations as in Example 1 were carried out to obtain 122.46 g of a copolymer $T_3$. The properties of the obtained copolymer $T_3$ were as shown in Table 4.

[Example 8]

Except for using 6.88 g (30 mmol) of the CBMA, 0.72 g (10 mmol) of the AA, 5.95 g (60 mmol) of the DMAA, and purified water (98.49 g), the same operations as in Example 1 were carried out to obtain 135.47 g of a copolymer $T_4$. The properties of the obtained copolymer $T_4$ were as shown in Table 4.

[Example 9]

Except for using 5.59 g (20 mmol) of the SBMA in place of the CBMA, 0.72 g (10 mmol) of the AA, 6.94 g (70 mmol) of the DMAA, and purified water (99.22 g), the same operations as in Example 1 were carried out to obtain 132.47 g of a copolymer $T_5$. The properties of the obtained copolymer $T_5$ were as shown in Table 4.

[Example 10]

Except for using 8.38 g (30 mmol) of the SBMA in place of the CBMA, 0.72 g (10 mmol) of the AA, 5.95 g (60 mmol) of the DMAA, and purified water (115.44 g), the same operations as in Example 1 were carried out to obtain 150.50 g of a copolymer $T_6$. The properties of the obtained copolymer $T_6$ were as shown in Table 4.

[Example 11]

Except for using 5.59 g (20 mmol) of the SBMA in place of the CBMA, 1.44 g (20 mmol) of the AA, 5.95 g (60 mmol) of the DMAA, and purified water (96.78 g), the same operations as in Example 1 were carried out to obtain 129.77 g of a copolymer $T_7$. The properties of the obtained copolymer $T_7$ were as shown in Table 4.

[Comparative Example 9]

Except for using 5.59 g (20 mmol) of the SBMA in place of the CBMA, 2.88 g (40 mmol) of the AA, 3.97 g (40 mmol) of the DMAA, and purified water (91.91 g), the same operations as in Example 1 were carried out to obtain 124.35 g of a copolymer $T_{C3}$. The properties of the obtained copolymer $T_{C3}$ were as shown in Table 4.

[Comparative Example 10]

Except for using 2.16 g (30 mmol) of the AA, 6.94 g (70 mmol) of the DMAA, and purified water (61.91 g), the same operations as in Example 1 were carried out to obtain 91.02 g of a copolymer $T_{C4}$. The properties of the obtained copolymer $T_{C4}$ were as shown in Table 4.

[Table 4]

|  | Copolymer T | Constituent monomer | Polymerization molar ratio | Mw | Mw/Mn |
|---|---|---|---|---|---|
| Example 7 | Copolymer $T_3$ | CBMA/AA/DMAA | 20/10/70 | 229k | 2.04 |
| Example 8 | Copolymer $T_4$ | | 30/10/60 | 177k | 1.73 |
| Example 9 | Copolymer $T_5$ | SBMA/AA/DMAA | 20/10/70 | 354k | 2.25 |
| Example 10 | Copolymer $T_6$ | | 30/10/60 | 159k | 1.59 |
| Example 11 | Copolymer $T_7$ | | 20/20/60 | 188k | 2.68 |
| Comparative Example 9 | Copolymer $T_{C3}$ | | 20/40/40 | 73k | 2.1 |
| Comparative Example 10 | Copolymer $T_{C4}$ | AA/DMAA | 30/70 | 61k | 2.37 |

[Examples 12 to 16]

Except for using the copolymer T shown in Table 5 in place of the copolymer $T_1$, the same operations as in Example 3 were carried out to obtain coated medical devices.

[Examples 17 to 21]

Except for using the copolymer T shown in Table 5 in place of the copolymer $T_1$, the same operations as in Example 5 were carried out to obtain coated medical devices.

[Comparative Examples 11 and 12]

Except for using the copolymer T shown in Table 5 in place of the copolymer $T_1$, the same operations as in Example 3 were carried out to obtain coated medical devices.

[Comparative Examples 13 and 14]

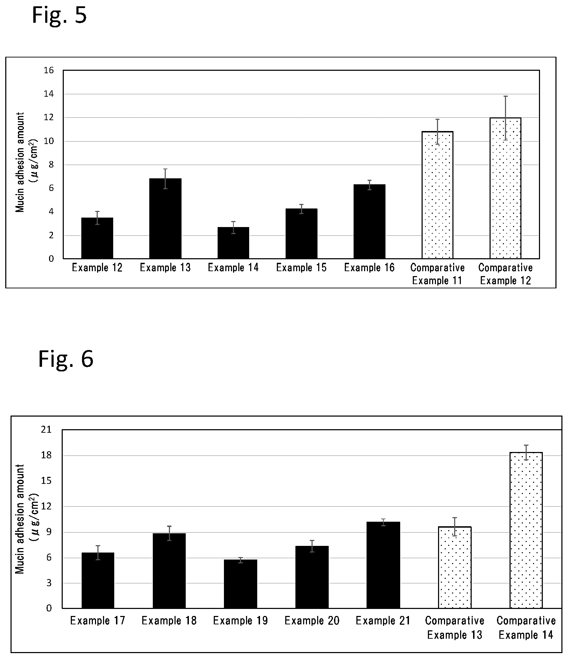

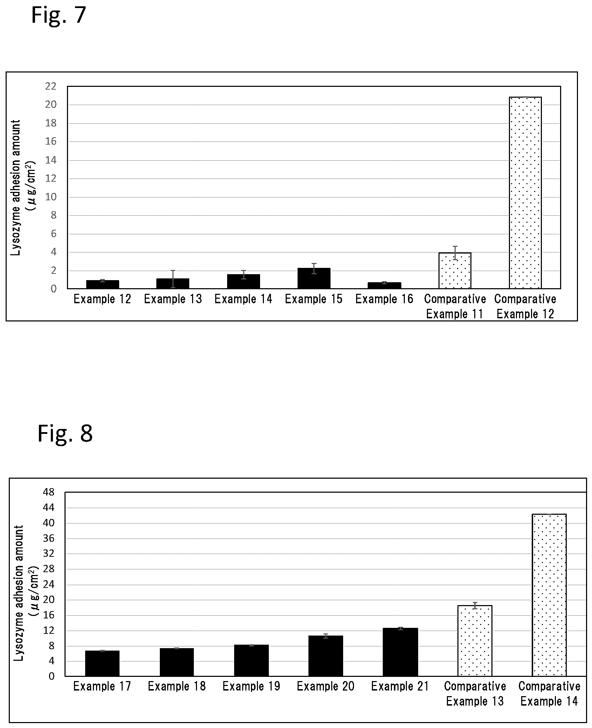

Except for using the copolymer T shown in Table 5 in place of the copolymer $T_1$, the same operations as in Example 5 were carried out to obtain coated medical devices.
The evaluation results for each of the coated medical devices obtained in Examples 12 to 21 and Comparative Examples 11 to 14 are shown in Table 5 and Figs. 5 to 8.

[Table 5]

| | Copolymer T | Liquid film retention time (second) | Liquid film retention time 2 hours after scrubbing and washing (sec) | Lubricity | Friction coefficient | Lipid adhesion |
|---|---|---|---|---|---|---|
| Example 12 | Copolymer $T_3$ | A (120) | A (67) | A | 0.122 | No adhesion |
| Example 13 | Copolymer $T_4$ | A (109) | A (32) | A | 0.133 | Partial adhesion |
| Example 14 | Copolymer $T_5$ | A (120) | A (95) | A | 0.112 | No adhesion |
| Example 15 | Copolymer $T_6$ | A (120) | A (55) | A | 0.129 | Partial adhesion |
| Example 16 | Copolymer $T_7$ | A (120) | A (65) | A | 0.147 | Partial adhesion |
| Example 17 | Copolymer $T_3$ | A (120) | A (97) | B | 0.19 | No adhesion |
| Example 18 | Copolymer $T_4$ | A (120) | A (91) | B | 0.227 | No adhesion |
| Example 19 | Copolymer $T_5$ | A (120) | A (B9) | B | 0.262 | No adhesion |
| Example 20 | Copolymer $T_6$ | A (120) | A (43) | B | 0.299 | No adhesion |
| Example 21 | Copolymer $T_7$ | A (20) | A (49) | B | 0.289 | No adhesion |
| Comparative Example 11 | Copolymer $T_{C3}$ | A (120) | A (120) | B | 0.165 | Partial adhesion |
| Comparative Example 12 | Copolymer $T_{C4}$ | A (20) | C (5) | A | 0.108 | Partial adhesion |
| Comparative Example 13 | Copolymer $T_{C3}$ | A (46) | A (76) | B | 0.37 | No adhesion |
| Comparative Example 14 | Copolymer $T_{C4}$ | A (120) | A (120) | A | 0.199 | No adhesion |

[Example 22]

As a medical device D, a stent made of a silicone (Dumon stent) was used. Except for using the medical device D in place of the medical device A, the same operations as in Example 12 were carried out to obtain a coated medical device.

[Example 23]

Except for using the medical device D in place of the medical device A, the same operations as in Example 14 were carried out to obtain a coated medical device.

[Comparative Example 15]

Except for using the medical device D in place of the medical device A, the same operations as in Comparative Example 12 were carried out to obtain a coated medical device.

[Comparative Example 16]

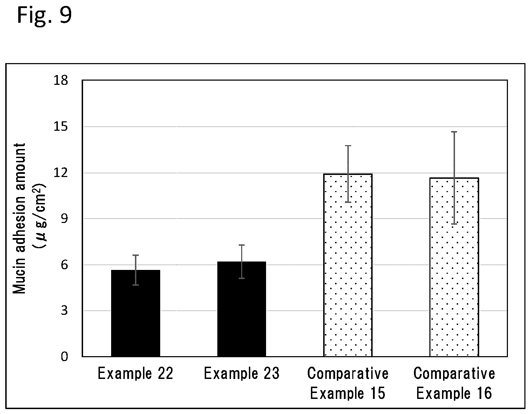

For the medical device D, the mucin adhesion amount was evaluated without performing the coating step.
The results of evaluating the mucin adhesion amount for each of the coated medical devices obtained in Examples 22 and 23 and Comparative Examples 15 and 16 are shown in Fig. 9.

## Claims

1.  A copolymer comprising the following (A), (B), and (C) as monomer components:

    (A) a compound having a carboxybetaine group and/or a sulfobetaine group;
    (B) a (meth)acrylic acid; and
    (C) an acrylamide derivative.

2.  The copolymer according to claim 1, wherein a content ratio of the monomer component (A) is 1 to 40 mol%.

3.  The copolymer according to claim 1 or 2, wherein a content ratio of the monomer component (B) is 1 to 35 mol%.

4.  A medical composition comprising the copolymer according to claim 1 and a solvent.

5.  A coated medical device, wherein a surface of a medical device is coated with the copolymer according to claim 1.

6.  A method for producing a copolymer, comprising a polymerization step of polymerizing a monomer containing (A) a compound having a carboxybetaine group and/or a sulfobetaine group, (B) a (meth)acrylic acid, and (C) an acrylamide derivative to obtain the copolymer.

7.  A method for producing a medical composition, comprising a preparation step of mixing the copolymer obtained by the method according to claim 6 and a solvent to obtain the medical composition.

8.  A method for producing a coated medical device, comprising a coating step of bringing the medical composition obtained by the method according to claim 7 into contact with a surface of a medical device to obtain the coated medical device.

9.  The method for producing a coated medical device according to claim 8, wherein the medical device is selected from the group consisting of an ophthalmic lens, a skin covering material, a wound covering material, a skin protective material, a drug carrier for skin, an infusion tube, a gas transport tube, a drainage tube, a blood circuit, a covering tube, a catheter, a stent, a sheath biosensor chip, a heart-lung machine, and an endoscope covering material.

10. The method for producing a coated medical device according to claim 9, wherein the ophthalmic lens is a contact lens.

Fig. 1

Fig. 2

Fig. 3

Fig. 4

Fig. 5

Fig. 6

Fig. 7

Fig. 8

Fig. 9

## INTERNATIONAL SEARCH REPORT

| International application No. |
| --- |
| **PCT/JP2023/014354** |

**A.    CLASSIFICATION OF SUBJECT MATTER**

*C08F 222/38*(2006.01)i; *A61L 15/24*(2006.01)i; *A61L 27/34*(2006.01)i; *A61L 29/08*(2006.01)i; *A61L 31/10*(2006.01)i; *C08F 220/06*(2006.01)i; *C08F 220/54*(2006.01)i; *G02C 7/04*(2006.01)i
FI:   C08F222/38; A61L15/24 100; A61L27/34; A61L29/08 100; A61L31/10; C08F220/06; C08F220/54; G02C7/04

According to International Patent Classification (IPC) or to both national classification and IPC

**B.    FIELDS SEARCHED**

Minimum documentation searched (classification system followed by classification symbols)

C08F222/38; A61L15/24; A61L27/34; A61L29/08; A61L31/10; C08F220/06; C08F220/54; G02C7/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C.    DOCUMENTS CONSIDERED TO BE RELEVANT**

| Category* | Citation of document, with indication, where appropriate, of the relevant passages | Relevant to claim No. |
| --- | --- | --- |
| X | CN 107200811 A (FUDAN UNIVERSITY) 26 September 2017 (2017-09-26) claims, paragraph [0010], examples | 1-9 |
| A | | 10 |
| X | CN 108676118 A (LEI, Zhouyue) 19 October 2018 (2018-10-19) claims, examples | 1-4, 6, 7 |
| A | | 5, 8-10 |
| X | CN 113185630 A (SICHUAN CHUANQING JINGXIA TECHNOLOGY CO., LTD.) 30 July 2021 (2021-07-30) claims, examples | 1-3, 6 |
| A | | 4, 5, 7-10 |

☐ Further documents are listed in the continuation of Box C.      ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **19 June 2023** | **27 June 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

PCT/JP2023/014354

| Patent document cited in search report | | | Publication date (day/month/year) | Patent family member(s) | Publication date (day/month/year) |
|---|---|---|---|---|---|
| CN | 107200811 | A | 26 September 2017 | (Family: none) | |
| CN | 108676118 | A | 19 October 2018 | (Family: none) | |
| CN | 113185630 | A | 30 July 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013024801 A
- WO 2014520191 A
- JP 4961133 B